Europäisches Patentamt

European Patent Office

Office européen des brevets

(11) Publication number: **0 285 578 B1**

(12) EUROPEAN PATENT SPECIFICATION

(45) Date of publication of patent specification: **01.09.93**  (51) Int. Cl.⁵: **G01N 27/416**

(21) Application number: **88830119.9**

(22) Date of filing: **25.03.88**

(54) Improvement of electrochemical devices for measuring the silicon content of hot metal.

(30) Priority: **27.03.87 IT 4777787**
**16.03.88 IT 4773488**

(43) Date of publication of application:
**05.10.88 Bulletin 88/40**

(45) Publication of the grant of the patent:
**01.09.93 Bulletin 93/35**

(84) Designated Contracting States:
**AT BE CH DE ES FR GB GR LI LU NL SE**

(56) References cited:
**EP-A- 0 208 072**
**US-A- 4 639 304**

**PATENT ABSTRACTS OF JAPAN, vol. 9, no. 227 (P-388)[1950], 13th September 1985;& JP-A-60 85 361**

**PATENT ABSTRACTS OF JAPAN, vol. 9, no. 265 (P-399)[1988], 23rd October 1985;& JP-A-60 113 145**

(73) Proprietor: **CENTRO SVILUPPO MATERIALI S.p.A.**
**P.O. Box 10747 Via di Castel Romano 100-102**
**I-00129 Roma(IT)**

Proprietor: **ILVA S.p.A.**
**Viale Castro Pretorio 122**
**I-00182 Roma(IT)**

(72) Inventor: **Granati, Paolo**
**Via Barbiellini Amidei, 97**
**Rome(IT)**
Inventor: **Palchetti, Maurizio**
**Via Clarice Tartufari n. 94**
**Rome(IT)**
Inventor: **Palella, Santi**
**Via Efestione, 16**
**Rome(IT)**
Inventor: **Federico, Giuseppe c/o Italsider SpA**

**Taranto(IT)**
Inventor: **Mazzarano, Andrea**
**Via Pisino, 155**
**Rome(IT)**

(74) Representative: **Gervasi, Gemma, Dr.**
**NOTARBARTOLO & GERVASI Srl 33, Viale Bianca Maria**
**I-20122 Milano (IT)**

## Description

The present invention relates to a concentration cell for measuring the silicon content of **hot** metal. More precisely, it concerns improvement of the concentration cells used for performing, on a continuous basis and for extended periods of time, silicon content measurements of the **hot** metal flowing through the blast-furnace main trough.

In the integrated iron and steel industry, iron ore is first reduced to hot metal in the blast furnace and subsequently refined to steel in the converter; this process is characterized today by an increasing tendency to use the converter exclusively for decarbonization purposes and under highly standardized operating conditions. As a result, other refining operations traditionally carried out in the converter have to be performed elsewhere.

The operations of desulphurization, desiliconization and dephosphorization, in particular, are competitive with each other and with the decarbonization reaction when they are performed in the converter. Under these conditions it is almost impossible to maintain the sulphur, silicon and phosphorus contents below the limits specified for the new "extra-clean" steels; in addition, blowing has to be continued for a longer period of time than is required for the sole elimination of carbon, with consequent waste of time and oxygen and an excessive oxidation of the metal bath.

For this reason, a number of proposals have already been made for treating the **hot metal** before it is loaded into the converter so as to obtain charges with controlled low sulphur and phosphorus contents; for example less than 0.01% S and less than 0.03% P.

One method proposed by the applicants for this patent refers to continuous dephosphorization of the hot metal flowing down the runners into the torpedo car.

Dephosphorization is slowed down to a considerable extent, however, when the silicon content of the hot metal is greater than 0.20% in weight. Since the same low-cost reactants (iron oxide and calcium oxide are used for lowering both the silicon and and the phosphorus content, this problem could be solved by adding the reactants in excess so as to desiliconize and dephosphorize at the same time.

Due consideration must be given, however, to the fact that dephosphorization does not proceed at the required rate, nor does it reach the desired intensity, unless a continuous effective contact is maintained between reactants and hot metal. In addition, since the output of a modern high-production blast furnace is more than 8000 ton of hot metal per day, any wasteful use of the reactants would (despite their low cost) inevitably involve far from negligible economic losses. Finally, it is not possible to operate with an excess of lime, since the basicity index of the slag (i.e. the ratio between the CaO and $SiO_2$ contents) must be kept around 5.

It is necessary therefore to measure the silicon content of the hot metal so as to determine whether desiliconizing is or is not required and, in the affirmative, how much reactant should be added to the melt.

Several methods have been developed for this purpose, but none of them is entirely satisfactory from the technical standpoint. In fact, the various possible solutions to the problem of determining the silicon content of **hot metal** fall into one of the following three main categories:

- Chemical analysis: analysis in the laboratory of a sample taken from the **hot** metal. This method gives very accurate results, but cannot be used for on-line continuous measurements owing to the time required for obtaining the determination.

- Thermochemical analysis: the method consists in determining with suitable measuring devices the cooling curve of a sample taken from the **hot** metal. The composition of the hot metal can be determined on the basis of the cooling rate measured under controlled conditions and of the variations that the cooling rate undergoes at specific temperatures. However, the sample is not a pure Fe-Si alloy and the influence of the silicon can be masked by the many other elements present in the hot metal The method can therefore give inaccurate results and, in addition, is too time-consuming to be useful for on-line continuous measurements.

- Electrochemical analysis: in this case, a concentration cell immersed in the **hot metal** measures the potential difference between a known reference Si activity $a_{Si\ (ref.)}$ and the activity of the unknown Si content of the hot metal $a_{Si\ (Fe)}$. The electromotive force E produced by the cell is given by the relationship

$$E = - \frac{RT}{nF} \ln \frac{a_{Si\ (Fe)}}{a_{Si\ (ref.)}}$$

2

where:

T = absolute temperature in °K

R = gas constant (8,3143 JK$^{-1}$ mol$^{-1}$)

F = Faraday's constant (96487 JV$^{-1}$mol$^{-1}$)

n = number of electrochemical equivalents.

At present, the last technique appears to be the most promising, since theoretically it offers the possibility of obtaining extremely accurate and sensitive measurements. As far as we know, however, the practical applications of the electrochemical technique made to date (presumably at laboratory level) do not meet the reliability and endurance requirements for operating immersed in hot metal.

A description of one of the early types of these concentration cells can be found in Transactions ISIJ, vol. 24, 1984, page B-45. It is stated in the article that the analysis must be completed in a very short time to ensure satisfactory silicon control, since the Si content of hot metal undergoes extremely rapid variations during tapping.

The proposed concentration cell consists of a hollow quartz tube, pierced at the end that is to be immersed in the **hot** metal and containing, just above the small perforation, a layer of silicate slag (that acts as an electrolyte) bearing a superimposed layer of metallic silicon. A graphite electrode is introduced in the tube so that its lower end is immersed in the slag layer; the upper end of the electrode is connected to a measuring device by a molybdenum wire conductor. The circuit through the **hot** metal is made, via the measuring instrument, by a second graphite electrode immersed in the **hot metal** nearby the quartz tube.

The measuring device just described exhibits good sensitivy and accuracy for short periods of operation, but has certain serious disadvantages.

In the first place, the graphite electrode immersed in the silicate slag tends, after a time, to dissolve into the slag; as a result, electrical conduction of the slag switches from ionic to electronic, short-circuiting the cell so that the latter ceases to operate. Secondly, after some time, the silicate slag starts to flow from the small hole at the end of the tube owing to capillary effects; in fact, the **hot metal** does not wet the quartz and the shallow depth at which the tube is immersed in hot metal does not provide a sufficient head to curb the capillarity forces. It follows that the cell empties itself and ceases to function long before the tapping operation has been completed.

A later partially improved measuring device of this type is described in Steel Research, 57 (4), 1986, pp. 166-171. In this case, a nickel-silicon alloy (saturated in nickel) is used as known reference instead of pure silicon (activity = 1) and the graphite electrode is replaced by a nickel wire electrode.

It is possible, with these modifications, to prevent degradation of the cell owing to dissolution of the nickel electrode, but other problems cannot be avoided. Firstly, the operating temperature of the device is limited to less than 1455°C, i.e. to the melting temperature of nickel. Secondly it is necessary to operate within a very narrow temperature range, given the wide gap between the liquidus and solidus phase equilibrium composition lines of the Ni-Si alloy; in fact, wide swings of the operating temperature would cause marked variations of the liquid composition (until the solid phase is also present) accompanied by variations of the electromotive force produced by the cell.

These operating temperature limitations render this particular device unsuitable for carrying out measurements in **hot metal** during tapping, since tapping temperatures range typically from 1470°C to 1530°C. Finally, this type of cell also tends to empty itself completely.

Even if it were possible to remove the operating temperature limitations by using other alloys (e.g. Fe-Si), it would still appear impossible to prevent the slag escaping from the device, at least with the designs proposed so far for the concentration cell.

Experimental tests carried out by us have shown that the cell empties itself in a few minutes, despite all attempts to improve the situation by adopting different cell geometries or by reducing the diameter of the hole which is necessary to ensure contact of hot metal with the slag acting as electrolyte.

The purpose of this invention is to propose an improved concentration cell capable of operating immersed in the **hot metal** flowing out of the blast furnace into the trough, for a duration of at least two hours, i.e. the time normally required for completing one of the tappings.

According to the invention, a concentration cell consisting of a tube of refractory material, preferably quartz, capable of withstanding high temperatures and slag attack (especially silicate slag) and containing: (i) a layer of slag which acts as an electrolyte and on which floats a layer of silicon that provides the silicon activity reference; (ii) an infusible electrode dipping into both the silicon and the slag layer, is improved by (a) separating the above-mentioned slag layer from the hot metal by means of a porous inert septum incapable of being fused by either **hot metal** or slag, and (b) using a silicon carbide electrode as the above-mentioned infusible electrode.

The porosity of the above-mentioned septum should be in the 100-350µm range, measured according to ATSM Specification D2440 XI. Preferably the porous septum should be of quartz.

The invention will now be described in greater detail with reference to the attached drawing which reproduces a non-limitative example of the invention.

In the drawing:

- Figure 1 shows a vertical cross-section of the cell that incorporates the improvements under this invention;
- Figure 2 shows schematically the resulting measuring device installed and ready for operation.

In both Figures, each component part is identified by the same reference number.

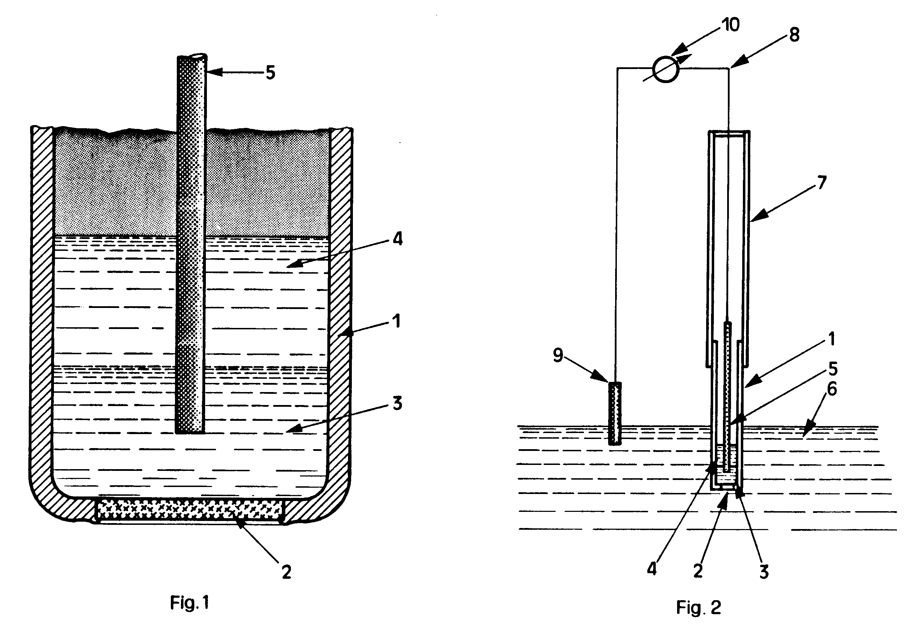

With reference to Figure 1, a hollow tube (1) made of refractory material, preferably quartz, is fitted with a porous septum (2), preferably also of quartz, inserted in the lower end of the tube. Within the tube, a layer of silicate slag (3) is placed just above the porous septum (2) and supports a layer of metallic silicon (4). An electrode (5) made of silicon carbide is inserted in the tube (1) so that its lower end passes through the metallic silicon layer (4) and dips into the underlying slag layer (3).

With reference to Figure 2, the electrochemical cell just described is shown mounted in a supporting framework (7) and immersed in a bath of hot metal (6). In due time, both slag (3) and metallic silicon (4) melt; the slag then becomes conductive and, as a result, current flows between the hot metal (of unknown silicon content) and the metallic silicon (4) contained in the tube (1) (activity = 1). A conductor (8), for example a molybdenum wire, connects electrode (5) to electrode (9), which latter is immersed in the hot metal and which, for example, is made of graphite. A measuring instrument (10) indicates the electromotive force E generated by the cell.

A number of electrochemical cells were prepared according to the above design, using porous septums made by the Heraeus Company and having different porosities (100, 160, 200, 290 and 350 µm).

These cells were first laboratory tested under controlled conditions and then tried out in the cast house for determining the Si content of the hot metal tapped from the blast furnace and flowing through the trough.

To ensure optimal execution of the industrial trials, the cells were preventively conditioned in a static bath of hot metal for periods of time varying from 15 to 30 minutes. As soon as the iron skull was burnt through, the pre-conditioned cell was immersed in the iron flowing into the trough and reading of the electromotive force was commenced.

Simultaneously with each reading, a sample was taken from the hot metal, in the close proximity to the sensing element of the cell, for chemical analysis in the laboratory of the silicon content.

The results obtained during the trials are given in Table I. In the table, the laboratory data are marked with an asterisk; each result represents the average of at least 10 measurements.

The stability of the cells was found to be excellent and in all trials the service life of the cell was always equal to, and sometimes greater than, the duration of one tapping operation, i.e. at least 90 minutes.

The instrument dead time between successive indications of hot metal composition variations was of the order of 2-3 seconds.

Agreement between electrochemical measurements and chemical reference measurements was excellent and within the normal experimental error range. Experimented septum porosity values were found to have practically no influence at all on the measurements.

## T A B L E   I

| EMF (mv) | 453 | 440* | 429* | 418 | 420 | 398* | 390 | 384 | 376 | 374 | 369 | 352* | 348* | 341* | 338 | 334 |
|---|---|---|---|---|---|---|---|---|---|---|---|---|---|---|---|---|
| Si % weight | 0,041 | 0,060 | 0,081 | 0,106 | 0,099 | 0,130 | 0,202 | 0,243 | 0,298 | 0,308 | 0,363 | 0,485 | 0,606 | 0,735 | 0,794 | 0,906 |

In a second embodiment of the present invention, the reference electrode with known silicon activity is obtained using a multiphase alloy or a compound, herein referred to as composition, of silicon with at least one other element having melting point higher than 1600°C, said element being chosen between Mo, Cr, Nb, Ta, V, W. Such alloy or compound shall be solid and with constant Si activity at the temperature of the testing.

4

EP 0 285 578 B1

In the cells according to this second embodiment, the infusible conductor is made of the same metal which forms with silicon the reference electrode.

A number of cells as shown in the drawings were prepared using as reference electrode the Mo-Si compound having composition $Mo_3 Si$, in presence of Molybdenum.

Such cells were tested in laboratory conditions, using hot metal baths with known Si contents and known temperatures.

The results obtained are in the following Table II, in which each EMF value is the average of at least 10 measurements.

T A B L E  II

| EMF (mv) | 165 | 140 | 123 | 98 | 82 | 70 | 48 | 35 |
|---|---|---|---|---|---|---|---|---|
| Si (% wgt) | 0.038 | 0.11 | 0.14 | 0.27 | 0.33 | 0.48 | 0.65 | 0.90 |

Also in this case agreement between electrochemical measurements and chemical reference measurements was excellent.

**Claims**

1. Concentration cell for measuring the silicon content of hot metal, said cell consisting of a tube of refractory material capable of withstanding high temperatures and slag attack (expecially silicate slag), and containing (i) a layer of slag acting as electrolyte, (ii) a superimposed metallic conductive layer acting as silicon activity reference, and (iii) an infusible electrode immersed in both the slag layer and the metallic conductive layer, characterized in that the above-mentioned slag layer is separated from the hot metal bath by means of a porous inert septum incapable of being fused by either hot metal or slag, said porous inert septum having a porosity ranging between 100 and 300 $\mu$m to permit to the said slag layer remaining in contact with the hot metal through the minute channels forming the porosity of the septum.

2. Concentration cells as under claim 1, characterized by the fact that the porous inert septum is made of quartz.

3. Concentration cell as under claim 1, characterized by the fact that said superimposed metallic conductive layer is made of silicon and said infusible electrode is made of silicon carbide.

4. Concentration cell as under claim 1, characterized in that said superimposed metallic conductive layer is made of a composition of silicon with at least one element having a melting point higher than 1600 °C, said element being chosen between Mo, Cr, Nb, Ta, V, W, and said infusible electrode being made of the same metal which forms with silicon said superimposed metallic conductive layer, and said composition having constant silicon activity at the temperature of testing.

**Patentansprüche**

1. Konzentrationszelle zum Messen des Silicumgehaltes heißen Metalls, wobei diese Zelle aus einem Rohr aus feuerfestem Material besteht, das in der Lage ist, hohen Temperaturen und Schlackenangriff (insbesondere von Silicatschlacke) standzuhalten, und (i) eine Schicht von als Elektrolyt wirkender Schlacke, (ii) eine überlagerte metallische leitfähige Schicht, die als Siliciumaktivitätsbezug wirkt und (iii) eine nicht schmelzbare Elektrode aufweist, die sowohl in die Schlackenschicht wie die metallische leitfähige Schicht eintaucht, **dadurch gekennzeichnet**, daß die oben genannte Schlackenschicht vom heißen bzw. schmelzflüssigen Metallbad vermittels eines porösen inerten Septums getrennt ist, das weder durch das heiße Metall noch durch die Schlacke geschmolzen werden kann, wobei dieses poröse inerte Septum eine Porosität, die zwischen 100 und 300 $\mu$m liegt, hat, um es dieser Schlakkenschicht zu ermöglichen, in Kontakt mit dem heißen Metall durch die winzigen, die Porosität des Septum bildenden Kanäle zu verbleiben.

2. Konzentrationszellen nach Anspruch 1, dadurch gekennzeichnet, daß das poröse inerte Septum (Trennwand) aus Quarz besteht.

3. Konzentrationszelle nach Anspruch 1, dadurch gekennzeichnet, daß diese überlagerte metallische leitfähige Schicht aus Silicium gemacht ist und diese nicht schmelzfähige Elektrode aus Siliciumcarbid gemacht ist.

4. Konzentrationszelle nach Anspruch 1, dadurch gekennzeichnet, daß diese überlagerte metallische leitfähige Schicht aus einer Siliciumzusammensetzung bzw. -verbindung gemacht ist, wobei wenigstens ein Element einen Schmelzpunkt höher als 1600°C hat, wobei dieses Element ausgewählt ist aus Mo, Cr, Nb, Ta, V, W und diese nicht schmelzfähige Elektrode aus dem gleichen Metall hergestellt ist, das mit dem Silicium diese überlagerte metallische leitfähige Schicht bildet und diese Zusammensetzung eine konstante Siliciumaktivität bei der Testtemperatur hat.

**Revendications**

1. Pile de concentration pour la mesure de la teneur en silicium de métal chaud, ladite pile se composant d'un tube en matière réfractaire capable de résister aux hautes températures et à l'attaque par les scories (en particulier les scories de silicate) et contenant: (i) une couche de scorie servant d'électrolyte, (ii) une couche métallique conductrice superposée, servant de référence d'activité de silicium, et (iii) une électrode infusible, immergée à la fois dans la couche de scorie et dans la couche métallique conductrice, caractérisée en ce que ladite couche de scorie est séparée du bain de métal chaud au moyen d'un diaphragme inerte poreux non susceptible d'être fondu par le métal chaud ou par la scorie, ce diaphragme inerte poreux ayant une porosité s'échelonnant entre 100 et 300 $\mu$m, pour permettre à ladite couche de scorie de rester en contact avec le métal chaud par les canaux minuscules formant la porosité du diaphragme.

2. Pile de concentration selon la revendication 1, caractérisée en ce que le diaphragme inerte poreux est fait de quartz.

3. Pile de concentration selon la revendication 1, caractérisée en ce que ladite couche métallique conductrice superposée est faite de silicium et ladite électrode infusible est faite de carbure de silicium.

4. Pile de concentration selon la revendication 1, caractérisée en ce que ladite couche métallique conductrice superposée est faite d'une composition de silicium avec au moins un élément ayant un point de fusion supérieur à 1600°C, cet élément étant choisi entre Mo, Cr, Nb, Ta, V, W, ladite électrode infusible étant faite du même métal qui forme, avec du silicium, ladite couche métallique conductrice superposée, et ladite composition ayant une activité de silicium constante à la température d'essai.

Fig. 2

Fig. 1